# EUROPEAN PATENT APPLICATION

(11) **EP 2 039 348 A1**
(43) Date of publication of application: **25.03.2009**
(21) Application number: 07018580.6
(22) Date of filing: 21.09.2007
(51) Int. Cl.: A61K 8/98, A61Q 19/00, A61Q 19/08, C12N 5/06, C12N 5/00, C12N 5/08

(54) **Cosmetic preparation and method to obtain a somatic stem cell preparation**

(71) Applicant: Schliefelbein, Jürgen, 35100 Maspalomas (ES)
(72) Inventor: Schliefelbein, Jürgen, 35100 Maspalomas (ES)
(74) Representative: Kailuweit & Uhlemann Patentanwälte

(57) **Abstract**

This invention concerns a cosmetic preparation containing somatic stem cells and a simple method to obtain a somatic stem cell preparation and its use, in particular to ameliorate the appearance of the skin.

In one embodiment the stem cells are isolated from human sternum in a simple, fast and painless way. This method advantageously makes an ambulant cosmetic or therapeutic treatment with stem cells in or even outside a hospital possible.

In another embodiment the stem cells are isolated from the animal bone marrow and preferably applied to the skin in form of a cream, salve, lotion, gel or wax.

## Description

This invention concerns a cosmetic preparation containing somatic stem cells and a simple method to obtain a somatic stem cell preparation and its use, in particular to ameliorate the appearance of the skin.

### State of the art:

Throughout a human life, the skin is exposed to many environmental factors, which often create stress and accelerate the skin's natural ageing process. The older we become, the more the elasticity of the skin is reduced, and wrinkles and other signs of ageing appear.

Known methods to reduce or smooth wrinkles include peeling methods and the injection of cosmetic fillers, like collagen, hyaluron acid, lactic acid or autologous adipose tissue. In most countries these methods can be applied by a cosmetician. Two other methods, which in most countries are only allowed to be carried out by a physician, are face lifting and the injection of botulinum toxin.

Stem cells are primal cells found in all multi-cellular organisms. They retain the ability to renew themselves through mitotic cell division and can differentiate into a diverse range of specialized cell types. The three broad categories of mammalian stem cells are: embryonic stem cells, derived from blastocysts, adult stem cells, which are found in adult tissues, and cord blood stem cells, which are found in the umbilical cord.

This invention only deals with somatic or adult stem cells. The term somatic or adult stem cell refers to any cell, which is found in a developed organism that has two properties: the ability to divide and create another cell like itself and the ability to divide and to create a cell more differentiated than itself. The term somatic stem cells is preferred over the term adult stem cells, as the cells can be found in children, as well as adults. Somatic stem cells are non-embryonic stem cells that are not derived from gametes (egg or sperm cells). Most somatic stem cells are lineage restricted (multipotent) and are generally referred to by their tissue origin (mesenchymal stem cell, adipose-derived stem cell, endothelial stem cell, etc.).

It is known to isolate somatic stem cells from the bone marrow of the iliac crest by surgery. However, this requires special needles, takes several hours, demands general anesthesia and has generally a painful outcome for the patients.

The somatic stem cells isolated by known techniques have to be enriched upon isolation by plating into tissue culture wells or by flow cytometry-based methods as sorting for specific surface markers. Plating into tissue culture wells has the disadvantage that a non-adherent stem cell population is lost. Culturing of the cells as well as enrichment by sorting can lead to contamination of the cells by allergenic substances or germs. Furthermore, culturing of stem cells is delicate, as the cells give easily rise to differentiated cells by loosing their stem cell characteristics.

### Objective:

A first objective of the invention is to provide a novel cosmetic preparation, in particular designed to ameliorate the appearance of the skin. Another objective of the invention is to provide a simple way to obtain a somatic stem cell preparation and its application.

### General Description of the Invention:

The first objective is solved by a cosmetic preparation containing somatic stem cells (also called adult stem cells).

The invention further refers to the use of somatic stem cells for cosmetic purposes, preferably for application to ameliorate the appearance of the skin, e. g. by improving the skin texture, in particular for the application to aged skin, in particular to crinkled, wrinkled and/or dimpled (cellulite) skin.

The somatic stem cells are preferably isolated from human or mammalian sternal bone marrow (breast bone), one preferred non-human mammal being the pig.

Advantageously; the cell preparation is only separated from red blood cells and serum by as simple means as centrifugation, no further enrichment by plating or cell sorting is needed.

Especially in the case that the cells are of human origin, autologous or autogenetic cells are preferred, meaning that the cells are isolated from the recipient himself or a genetically identical twin. The cells of human origin are preferably freshly isolated, meaning that they are freshly prepared and administered to a recipient without delay, meaning at least within 5 hours, preferably 2 hours, most preferably within 30 minutes upon isolation.

Alternatively, especially in case that the cells are of non-human origin, preferably from pigs, the cells are lyophilised upon isolation. Especially in this case the cosmetic preparation is preferably formulated as cream, lotion, gel or wax. To obtain the stem cells from animals, bones, especially the sterns (breast bones,) can be easily separated from the carcass and broken open to isolate the bone marrow. The bone marrow is separated from the remaining bones (e. g. by diluting and passing through a mesh) and preferably centrifuged to obtain a serum containing upper layer and a red blood cell containing lower layer. The intermediate layer (situated between the serum containing upper layer and the red blood cell containing lower layer) is withdrawn to obtain a stem cell enriched suspension. This suspension is freeze dried to obtain a powder.

The cosmetic preparation contains preferably as further component a local anesthetic, like Procaine, Tetracaine, Chloroprocaine, Benzocaine, Bupivacaine, Lidocaine, Mepivacaine, Prilocaine, Cinchocaine, Etidocaine, Articaine, Ropivacaine or Levobupivacaine, even when formulated as cream, lotion, gel or wax. These substances advantageously improve the cosmetic effect. The local anesthetic is preferably added in a concentration of 0.05 % to 0.5 %, preferably 0.1 to 0.3 % referring to the final volume.

The cosmetic preparation is preferably applied cutaneously, subcutaneously or percutaneously, either topically, transdermally, intradermally or interepidermally. For direct injection of the stem cells into the skin the cosmetic preparation is preferably an autologous (or autogenic) somatic stem cell suspension supplemented by growth factors isolated from peripheral blood of the recipient or a growth factor enriched autologous serum fraction. Advantageously the stem cell suspension can be applied as easy as cosmetic filler, known from the state of the art. The stem cell preparation is preferably injected in several spots into the area, where the skin texture is to be ameliorated, e. g. around and/or under the crinkled, wrinkled and/or dimpled skin preferably 200 µl to 2 ml, most preferably 0.5 ml to 2 ml per spot.

The second objective is solved by a method for the production of a somatic stem cell preparation with the following steps:
a. An isolated probe of bone marrow fluid (containing bone marrow cells), which is obtained from the sternum, is centrifuged to obtain a serum containing upper layer and a red blood cell containing lower layer, whereas the stem cells are recovered from the intermediate layer to form fraction A.
b. An isolated probe of peripheral blood is centrifuged to obtain a serum containing upper layer and a red blood cell containing lower layer, whereas the lower area (preferably the lower third or the lower 20 to 40 %) of the serum layer is recovered to form fraction B. As a result of this centrifugation step the lower area of the serum layer is enriched in growth factors.
c. Fractions A and B are mixed.

The preceding step, the isolation of the bone marrow cells from the sternum, can advantageously be achieved in a fast, painless way by simple aspiration with a standard needle, normally used for taking blood, and local anesthesia. Advantageously the needle used for application of the local anesthetic can be employed immediately for aspiration of the bone marrow fluid. After application of the local anesthetic the needle is preferably inserted deeper into the sternum for aspiration of the bone marrow fluid In this way one puncture can be sufficient for both, the application of the local anesthetic and the aspiration of the bone marrow fluid.

If more stem cells are needed the sternum can be punctured twice or even three times.

The centrifugation in step a. and b. can advantageously be carried out in a standard bench-top centrifuge in a gentle, simple way, within 1 to 10 minutes depending on the speed, which is preferably chosen within 500 to 4000 rpm. However, it is clear that a centrifugation to obtain a serum containing upper layer and a red blood cell containing lower layer, can be done under other conditions, like for instance by adding sterile density medium, like Ficoll-Paque^{®} (a non ionic synthetic polymer of sucrose) or other, and higher centrifugation times and speed. However, as these methods are more complicated and time consuming they are less preferred.

Advantageously no further purification, separation, cell culture, enrichment by cell-sorting or by plating is needed. The lack of these steps not only simplifies the isolation but avoids contamination and preserves the stem cell properties of the cells. No further cell culture or amplification is needed as the amount of stem cells obtained by this method is sufficient for the application, because of the lack of receptors in the recipient As the stem cell preparation is applied to the same individual, from whom it is isolated, and does not contain any harmful by-products it is very well tolerated. The individual in the context of this description is preferably a human being or another mammal.

The bone marrow fluid in step a. and the blood probe in step b. are preferably only supplemented with known additives preventing blood coagulation, preferably heparine in step a. and citrate in step b. These additives are preferably added directly after isolation from the human body.

Advantageously the above mentioned steps can be carried out within less than 5 hours, preferably within 2 hours or even 30 minutes, making it possible to reapply the stem cell preparation, preferably to the skin of the same individual, in the same sitting.

Accordingly a method of treatment wherein somatic stem cells are freshly isolated from the bone marrow of an individual (preferably the sternum) and reapplied to another organ, preferably the skin, of the same individual without delay, meaning within the same sitting or less than 5 hours, preferably within 2 hours, most preferably within 30 minutes, is also part of the invention. This method advantageously makes an ambulant treatment with stem cells in or even outside a hospital possible. For this method the stem cells are preferably isolated by the above mentioned steps a. to c.

The invention also comprises the somatic stem cell preparation produced by the above mentioned steps a. to c.. The stem cell preparation contains preferably 1 part of fraction A and 2 to 5 parts of fraction B, preferably 2.5 to 3.5 parts of fraction B. This corresponds to 1 ml to 8 ml, preferably 2 ml to 6 ml, of fraction A and 3 ml to 30 ml, preferably 5 ml to 15 ml, of fraction B.

The stem cell preparation obtained in step c. is preferably supplemented with a local anesthetic, like Procaine, Tetracaine, Chloroprocaine, Benzocaine, Bupivacaine, Lidocaine, Mepivacaine, Prilocaine, Cinchocaine, Etidocaine, Articaine, Ropivacaine or Levobupivacaine. The local anesthetic is preferably added in a concentration of 0.05 % to 0.5 %, preferably 0.1 % to 0.3 % referred to the final volume.

Advantageously no further supplements are needed.

Although the focus of this invention is the use of somatic stem cells for cosmetic purposes, a further aspect of the invention is the use of somatic stem cells isolated from the sternum for example prepared by the method described above, or somatic stem cells isolated from animal bone for therapeutic purposes, e. g. in a medicament. This therapeutic agent or the above mentioned method of treatment can be used for the treatment of cancer (e. g. of the brain, retina, breast, ovaries, skin, testicules, lymphoma, leukemia), auto-immune diseases (as Diabetes Type 1, Rheumatoid Arthritis, Multiple Sclerosis, Myasthenia) and immunodeficiencies (as Severe Combined Immunodeficiency Syndrome), Cardiovascular diseases (as Acute Heart Damage and Chronic Coronary Artery Disease), Neural Degenerative Diseases and Injuries (as Parkinson's Disease, Alzheimer's Disease, Spinal Cord Injury, Stroke Damage), anaemia, metabolic disorders, disorders in peripheral blood circulation, venous ulcers, ulcus cruris, liver failure and cirrhosis, bladder disease, scares, lesions, varicosities, wounds, injuries (like spinal cord injury) and for ocular regeneration.

A further object of the invention is the use of somatic stem cells isolated from the bone marrow of an individual, preferably the sternal bone marrow, to manufacture a therapeutic agent, whereas the therapeutic agent is reapplied (or prepared for reapplication) in the same sitting or at least within 5 hours, preferably within 2 hours, most preferably within 30 minutes, upon isolation. The therapeutic agent is preferably the autologous stem cell preparation described above, most preferably supplemented with a local anesthetic. As the therapeutic agent is prepared for reapplication without delay it does advantageously not contain any traces of cell culture medium or other by-products. The therapeutic agent is preferably used to treat the above mentioned indications.

Another aspect of the invention is the use of the somatic stem cell preparation according to the invention or the use of stem cells prepared from the sternum together with autologous blood in autohaemotherapy or for the manufacture of an agent for the use in autohaemotherapy. The autologous blood is preferably first treated by ozone and/or oxygen and then mixed with stem cells isolated from the sternum as described in step a. or a stem cell preparation as obtained in step c.. This mixture can be applied parenterally, e. g. by injection, infusion or implantation , to an individuaL

A further aspect of the invention is a cream, salve, lotion, gel or wax or another composition for topical administration containing somatic stem cells, preferably isolated from animal bone marrow, preferably from pigs. This composition can not only be used for cosmetic but also for therapeutic purposes, especially for the treatment of scares, lesions, varicosities, Rheumatoid Arthritis, disorders in peripheral blood circulation and venous ulcers, like ulcus cruris. The somatic stem cells are preferably added in lyophilized form to a known cream, salve, lotion, gel or wax formulation.

This cream, salve, lotion, gel or wax or pharmaceutical composition for topical administration contains preferably as further component a local anesthetic, like Procaine, Tetracaine, Chloroprocaine, Benzocaine, Bupivacaine, Lidocaine, Mepivacaine, Prilocaine, Cinchocaine, Etidocaine, Articaine, Ropivacaine or Levobupivacaine. The local anesthetic is preferably added in a concentration of 0.05 % to 0.5 %, preferably 0.1 % to 0.3 % referred to the final volume. The cream, salve, lotion, gel or wax is preferably applied topically (on the surface of the skin). However, its action can also be transdermally.

The invention is illustrated by the following examples without being limited to these:

### Example 1:

This example describes the isolation of stem cells from the human sternum and their cosmetic application.

First, the skin upon the sternum is cleaned with an antiseptic (like usually Betadine). Then a local anesthetic (e. g. 2% Lidocaine) is applied with a standard cannula (e. g. a 21 gauge 1/2 - with luer-lock), which is normally employed for taking blood samples, into the skin in the location of the sternum. Then the cannula is locked on a 10 ml-syringe prefilled with I ml Heparin-solution, and is inserted into the sternum, 5 to 6 ml of bone marrow fluid flows into the tube - further referred to as tube A. The needle is removed.

Further, 10 to 12 ml peripheral (venous) blood are taken from the same person, with a standard blood taking syringe (e. g. as described above) prefilled with 1 ml sodium citrate (10 %). The blood is evenly distributed onto two tubes - further referred to as tubes B.

Tube A and the tubes B are centrifuged at 1000 rpm in a standard bench-top centrifuge for 3 min, to obtain a serum containing upper layer and a red blood cell containing lower layer.

In tube A the intermediate layer (situated between the serum containing upper layer and the red blood cell containing lower layer) is carefully withdrawn using a pipette to obtain about 2 ml stem cell enriched fluid - further referred to as fraction A.

In each tube B the lower third of the upper layer (about 3 ml each) is carefully withdrawn using a pipette to obtain in total about 6 ml growth factor enriched fluid - further referred to as fraction B.

Fractions A and B are carefully mixed and supplemented with 2 ml 1 % Lidocaine to obtain 10 ml stem cell preparation, which is ready for the cosmetic application. If higher volumes are needed, the sternum can be punctured twice or three times - obtaining 5 to 6 ml of bone marrow fluid per puncture. The amount of peripheral blood taken is raised accordingly.

The surface of the skin in the area, where the stem cells shall be applied to, is disinfected. The stem cell preparation produced as described above is injected into several spots around and under the crinkled, wrinkled and/or dimpled skin (about 1 to 2 ml per spot). As the stem cell preparation is applied to skin of the same person, from whom it is isolated, and does not contain any harmful by-products it is very well tolerated. As the stem cells are prepared very fast they can be applied to the person within less than an hour, even within 30 minutes, upon isolation (in the same sitting) making an ambulant treatment possible.

### Example 2:

This example describes the isolation of stem cells from the sternum of pigs and their cosmetic application.

Sterna (breast bones) are taken from pig carcasses and broken open to isolate bone marrow. The bone marrow is suspended in phosphate buffer containing heparine, passed through a mesh and centrifuged at 1000 rpm for 5 min to obtain a serum containing upper layer and a red blood cell containing lower layer. The intermediate layer (situated between the serum containing upper layer and the red blood cell containing lower layer) is withdrawn to obtain a stem cell enriched suspension. This suspension is freeze dried to obtain a powder.

A cream is formulated by mixing the following ingredients:
a.) 1 part stem cell powder as prepared above,
b.) 4 parts glycerol monostearate 60,
c.) 6 parts cetyl alcohol,
d.) 7,5 parts capric triglyceride,
e.) 25,5 parts vaseline,
f.) 7 parts macrogol-1000-stearate,
g.) 10 parts propylene glycol,
h.) 5 g hydrogenated lecithin,
i.) 40 parts water.

Glycerol monostearate 60, cetyl alcohol, capric triglyceride and Vaseline are heated to 60 °C and added to a equi-tempered mixture of macrogol-1000-glycerol monostearate, propylene glycol and water. The cream is stirred while cooling down. When the temperature has reached 40 °C the stem cell powder is added.

The cream is applied to the areas of the skin of a person, where the texture shall be ameliorated, e. g. to the crinkled, wrinkled and/or dimpled skin.

## Claims

1. Use of somatic stem cells for cosmetic purposes, in particular for the amelioration of the appearance of the skin or the amelioration of the skin texture.

2. Cosmetic preparation containing somatic stem cells.

3. Use according to claim 1 or cosmetic preparation according to claim 2, wherein the stem cell preparation is applied to aged skin, in particular to crinkled, wrinkled and/or dimpled skin.

4. Use according to claim 1 or 3 or cosmetic preparation according to claim 2 or 3, wherein the stem cells are freshly prepared and administered to a recipient within 5 hours, preferably within 30 minutes, upon isolation.

5. Use according to one of the claims 1 or 3 to 4 or cosmetic preparation according to one of the claims 2 to 4, wherein the stem cells are prepared from human sternum or animal bone marrow.

6. Use or cosmetic preparation according to claim 5, wherein the stem cells are prepared from pig bone marrow.

7. Cosmetic preparation according to one of the claims 2 to 6, containing additionally growth factors isolated from peripheral blood or a growth factor enriched serum fraction.

8. Cosmetic preparation according to one of the claims 2 to 7, formulated as crème, lotion, gel, wax or salve.

9. A process for the production of a somatic stem cell preparation, wherein
a. bone marrow fluid containing bone marrow cells, which is isolated from the sternum, is centrifuged to obtain a serum containing upper layer and a red blood cell containing lower layer, whereas the stem cells are recovered from the intermediate layer to form fraction A,
b. an isolated probe of peripheral blood is centrifuged to obtain a serum containing upper layer and a red blood cell containing lower layer, whereas the lower area of the serum layer, which is enriched in growth factors, is recovered to form fraction B,
c. fractions A and B are mixed.

10. Somatic stem cell preparation obtained by the process according to claim 9, preferably containing 1 part of fraction A and 2 to 5 parts of fraction B.

11. Cream, salve, lotion, gel or wax containing somatic stem cells.

12. Cosmetic preparation or somatic stem cell preparation or cream, salve, lotion, gel or wax according to one of the above mentioned claims containing a local anesthetic as further component.

13. Use of somatic stem cells to manufacture a therapeutic agent, preferably a stem cell preparation according to claim 9 or 10, wherein the therapeutic agent is prepared for reapplication in the same sitting or within 5 hours, preferably within 30 minutes, upon isolation.
